# EUROPEAN PATENT APPLICATION

(11) **EP 4 035 733 A1**
(43) Date of publication of application: **03.08.2022**
(21) Application number: 20867214.7
(22) Date of filing: 24.09.2020
(51) Int. Cl.: A61P 3/00, A61K 9/16, A61K 47/26, A61K 47/38, A61K 31/315

(54) **GRANULE HAVING MASKED UNPLEASANT TASTE AND METHOD FOR PRODUCING SAME**

(30) Priority: 25.09.2019 JP 2019173744
(71) Applicant: Nobelpharma Co., Ltd., Chuo-ku Tokyo 104-0033 (JP)
(72) Inventor: NOMURA Tatsuo, Tokyo 104-0033 (JP); YASUZAWA Toru, Tokyo 104-0033 (JP)
(74) Representative: Schön, Christoph
(86) International application number: PCT/JP2020/035883
(87) International publication number: WO 2021/060304

(57) **Abstract**

Provided is a granule in which an unpleasant taste of an active ingredient is masked and immediate release of the active ingredient is maintained, and a method for producing the granule. In particular, provided is the granule containing zinc acetate as an active ingredient having an unpleasant taste, and a method for producing the granule.

A method for producing a granule in which an unpleasant taste of an active ingredient is masked and immediate release of the active ingredient is maintained. The method includes a step (1) of obtaining a core particle containing an active ingredient having an unpleasant taste, a step (2) of coating a surface of the core particle obtained in the step (1) with ethylcellulose to obtain a particle, and a step (3) of subjecting the particle obtained in the step (2) to a heat treatment in a closed state.

## Description

### Technical Field

The present invention relates to a granule in which an unpleasant taste of an active ingredient is masked and immediate release of the active ingredient is maintained, and to a method for producing the granule. In particular, the present invention relates to the granule containing zinc acetate as an active ingredient having an unpleasant taste and to a method for producing the granule.

### Background Art

In general, as in the phrase "good medicine tastes bitter", some drugs having an excellent therapeutic effect have a very unpleasant taste. For example, zinc acetate, which has been used as a therapeutic agent for Wilson disease and has recently been clinically applied as a therapeutic agent for zinc replacement therapy to hypozincemia patients, has a very unpleasant taste.

The therapeutic agents containing zinc acetate that have been used are a capsule or a film-coated tablet, and therefore the problem concerning the unpleasant taste of the active ingredient at the time of dosing has not been apparent. However, the zinc acetate preparations are used for patients of a wide age range from children to the elderly. Therefore, a granular preparation is awaited that is further easy to take and whose dose is easy to adjust.

Here, in a case where an active ingredient having a very unpleasant taste such as zinc acetate is used in a granule, the preparation dissolves in the mouth at the time of dosing, and the unpleasant taste of the active ingredient is directly felt, and therefore the unpleasant taste is to be masked. Meanwhile, in order to obtain the sufficient effect of the drug, the drug such as zinc acetate is to be absorbed into the living body at an early stage after the administration. Therefore, in order to use a drug such as zinc acetate in a granule, contradictory problems of masking the unpleasant taste and maintaining the immediate release are to be simultaneously overcome.

Patent Literature 1 discloses a method in which a core containing a pharmacologically active substance is coated with a solution obtained by dissolving ethylcellulose and hydroxypropylcellulose in hydrous ethanol so that the coating rate to the raw granule is 60 to 100% to produce a granular preparation in which the unpleasant taste is masked and the immediate release is achieved.

Patent Literature 2 discloses a method, as a method of coating without using an organic solvent, in which an ethylcellulose aqueous suspension is used. Patent Literature 2 discloses a method in which a core containing a pharmacologically active substance is coated with an ethylcellulose aqueous suspension, and then the resulting product is heat-treated under a certain humidity.

Patent Literature 3 discloses a method, as a technique for masking of an unpleasant taste and for rapid release, in which a core containing a pharmacologically active substance is first coated with a water-soluble polymer as an intermediate layer, and the intermediate layer is coated with an ethylcellulose aqueous suspension containing a certain amount of a plasticizer at a coating rate of 20 to 40%.

### Citation List

### Patent Literature

Patent Literature 1: JP 2008-81448 A
Patent Literature 2: JP H9-194347 A
Patent Literature 3: JP 2000-53563 A

### Summary of Invention

### Technical Problem

As described above, in a granule containing an active ingredient having an unpleasant taste such as zinc acetate, the contradictory problems of masking the unpleasant taste and maintaining the immediate release are to be simultaneously overcome.

However, the invention described in Patent Literature 1 to 3 has not provided a granule in which the immediate release is maintained while the unpleasant taste of the active ingredient is masked. The step of heating a granule is usually performed in an open system in order to avoid a positive pressure, but even this method has not provided a granule in which the immediate release is maintained while the unpleasant taste of the active ingredient is masked.

The present invention has been made in view of the above-described circumstances, and an object of the present invention is to provide a granule in which an unpleasant taste of an active ingredient is masked and immediate release of the active ingredient is maintained, and to provide a method for producing the granule.

### Solution to Problem

In order to solve the above-described problem, the present inventors have intensively studied a heat treatment in a coating step in a conventional open system. As a result, surprisingly, the present inventors have solved the problem by performing the heating step in a closed state against the common technical knowledge, and have reached the present invention. That is, by subjecting a core particle sprayed with a water-dispersed ethylcellulose suspension to a heat treatment in a closed state, a granule has been successfully obtained that has a coating in an amount or thickness such that after the dosing, the active ingredient having an unpleasant taste is not released in the mouth but after the swallowing, the active ingredient is immediately released in the stomach, and the present invention has been achieved.

In the present description, the term "core particle" is used to mean a particle that is a center of each particle included in a granule. The term "immediate release" is used to mean that after the swallowing, the active ingredient is released in the stomach at an early stage.

That is, the present invention is a method for producing a granule in which an unpleasant taste of an active ingredient is masked and immediate release of the active ingredient is maintained, and the method includes a step (1) of obtaining a core particle containing an active ingredient having an unpleasant taste, a step (2) of coating a surface of the core particle obtained in the step (1) with ethylcellulose to obtain a particle, and a step (3) of subjecting the particle obtained in the step (2) to a heat treatment in a closed state.

Furthermore, the present invention is the granule in which an unpleasant taste of an active ingredient is masked and immediate release of the active ingredient is maintained, obtained by the method according to the present invention.

Furthermore, the present invention is a granule including a core particle containing an active ingredient having an unpleasant taste and including a film containing ethylcellulose in an amount of 2 to 12 wt% with respect to the core particle, wherein the core particle is coated with the film, the unpleasant taste of the active ingredient is masked, and immediate release of the active ingredient is maintained.

That is, the present invention is a granule including a core particle containing an active ingredient having an unpleasant taste and including a film containing ethylcellulose in an amount of 2 to 12 wt% with respect to the core particle, wherein the core particle is coated with the film, and when the granule is measured with the Japanese Pharmacopoeia Dissolution Test Method 2 (Puddle Method) using 900 mL of water as a dissolution medium at 50 rpm, the dissolution rate of the active ingredient is 80% or less at a test time of 5 minutes, and 85% or more at a test time of 15 minutes.

### Advantageous Effects of Invention

According to the present invention, it is possible to provide a granule in which an unpleasant taste of an active ingredient is masked and immediate release of the active ingredient is maintained, and to provide a method for producing the granule. In particular, it is possible to provide the granule containing zinc acetate as an active ingredient having an unpleasant taste and to provide a method for producing the granule.

### Brief Description of Drawings

Fig. 1 is a graph showing a comparison between the dissolution rate of an active ingredient in a preparation heated under a closed condition and that in a preparation heated under a open condition.
Fig. 2 is a graph showing the relationship between the coating amount of ethylcellulose and the dissolution rate of an active ingredient.
Fig. 3 is a graph showing the relationship between the addition amount of a plasticizer and the dissolution rate of an active ingredient.

### Description of Embodiments

Hereinafter, the present invention will be described in detail.

Examples of an active ingredient that has an unpleasant taste and can be used in the present invention include various substances having an unpleasant taste such as bitterness and astringency, and highly water-soluble substances are particularly effectively used in which the immediate release has been difficult to maintain while the taste is masked. Examples of such a substance include zinc acetate. Zinc acetate may be an anhydride or a hydrate, and is more preferably a dihydrate.

In one aspect of the present invention, a step (1) can be a step of spraying a particle to be a core of a core particle with a solution or a suspension of the active ingredient to coat the particle with the active ingredient.

The particle to be a core of a core particle is not particularly limited as long as the particle contains a substance having a disintegrating property in the body such as a saccharide and/or crystalline cellulose as a main component. For example, particles can be used such as a particle containing sucrose (sucrose defined in the Japanese Pharmacopoeia) or sucrose and corn starch as main components, a particle including lactose and crystalline cellulose, a spherical particle including D-mannitol, and granulated sugar.

The shape of the particle to be a core of a core particle is not particularly limited, and a particle having a substantially spherical shape can be preferably used. As the shape of the particle is closer to a spherical shape, the coating with the active ingredient or the like is more uniform to have a more stable quality.

In one aspect of the present invention, the step (1) can be a step of coating the particle with the active ingredient and a binder. The binder may be contained in the solution or the suspension of the active ingredient and sprayed together with the active ingredient, or may be prepared as a solution other than the solution or the suspension of the active ingredient and sprayed simultaneously with the active ingredient or separately. If the particle to be a core is coated with the binder and the active ingredient, the bond between the active ingredient and the particle can be further strengthened.

The binder is to be a binder generally used in the pharmaceutical field, and examples of the binder include hydroxypropylcellulose, polyvinyl alcohol, hydroxypropylmethylcellulose, methylcellulose, gum arabic, gelatin, and starches, and hydroxypropylcellulose is preferable.

The blending amount of the binder is to be an amount such that the particle to be a core and the active ingredient can be sufficiently bonded to each other, and the blending amount can be appropriately set.

In one aspect of the present invention, the step (1) can be a step of coating the particle with the active ingredient and a disintegrant. The disintegrant may be contained in the solution or the suspension of the active ingredient and sprayed together with the active ingredient, or may be prepared as a solution other than the solution or the suspension of the active ingredient and sprayed simultaneously with the active ingredient or separately.

The disintegrant is to be a disintegrant generally used in the pharmaceutical field, and examples of the disintegrant include partly pregelatinized starch, carboxymethylcellulose, croscarmellose sodium, and crospovidone, and partly pregelatinized starch is preferable.

The amount of the disintegrant can be appropriately adjusted so as to achieve a desired dissolution speed.

In the present invention, the particle may be coated with the active ingredient and with the binder or the disintegrant, or may be simultaneously coated with the active ingredient, the binder, and the disintegrant. In a case where the particle is simultaneously coated with the binder and the disintegrant, the binder and the disintegrant may be blended in the solution or the suspension of the active ingredient and sprayed together with the active ingredient, or may be prepared as a solution of the binder and the disintegrant other than the solution or the suspension of the active ingredient and sprayed simultaneously with the active ingredient or separately.

In one aspect of the present invention, the step (1) can be a step of grinding a granulated product that includes the active ingredient having the unpleasant taste and a preparation aid such as an excipient to obtain particles, and sieving the particles to obtain particles having a certain particle size.

Examples of the excipient include saccharides such as lactose, sucrose, and mannitol, starches such as corn starch and potato starch, and binders such as hydroxypropylcellulose, methylcellulose, hydroxypropylmethylcellulose, polyvinylpyrrolidone, and polyvinyl alcohol, and particles obtained by granulation and sieving to 100 to 600 µm, preferably 200 to 500 µm according to a conventional method can be used as core particles having the active ingredient.

In one aspect of the present invention, the step (2) can be a step of spray-coating the core particle with a water-dispersed ethylcellulose suspension.

The coating amount of the ethylcellulose is 2 to 12 wt%, preferably 5 to 9 wt%, and more preferably 5 to 7 wt% with respect to the core particle.

In one aspect of the present invention, the step (2) can be a step of coating the core particle with ethylcellulose and a plasticizer. If coated with ethylcellulose and a plasticizer, the particle can be further completely coated with a film.

The plasticizer is to be a plasticizer generally used in the pharmaceutical field, and examples of the plasticizer include triethyl citrate, polyethylene glycol, propylene glycol, and triacetin.

The plasticizer can be used in an amount appropriately selected. For example, in the case of coating a granule having a particle size of 200 to 1,000 µm, the plasticizer is to be used in an amount of about 5 to 20 wt% with respect to ethylcellulose.

In the present invention, the heat treatment in the step (3) is a step of heating the particle in a closed state so that release of the moisture in the particle to the outside of the system due to overheating can be prevented to some extent. The phrase "heating the particle in a closed state" means to heat the particle under a closed condition, and the condition is to be a condition in which release of the moisture in the particle to the outside of the system can be prevented to some extent. For example, the phrase means to heat the particle obtained in the step (2) in a state of being put in a closed container. The heating in the step (3) is preferably performed under a so-called airtight condition, that is, a condition in which no liquids flow in and out, and more preferably performed in a so-called closed state, that is, under a condition in which no gases such as water vapor and air flow in and out. The heating condition is to be sufficient to form a film of ethylcellulose coating the particle surface, and for example, the temperature can be 70 to 100°C, and the treatment time can be 15 to 180 minutes.

In a preferable aspect, before the heating in the step (3), a glidant can be added to the particle obtained in the step 2. If a glidant is added, adhesion and aggregation of the particles can be prevented in the heating.

The glidant is to be a glidant generally used in the pharmaceutical field, and examples of the glidant include light anhydrous silicic acid.

The glidant is to be added in an amount sufficient to disperse the particles, and specifically, to be used in an amount of about 0.5 to 1 wt% with respect to the particles.

In the present invention, the phrase "an unpleasant taste of an active ingredient is masked and immediate release of the active ingredient is maintained" means that the granule has performance such that at the initial stage of the administration of the granule (time during which the granule is present in the mouth), the active ingredient is not dissolved and after a lapse of a certain period of time (at a time point in an early stage after the swallowing), a sufficient amount of the active ingredient is dissolved. For example, the granule has performance such that when the granule is measured with the Japanese Pharmacopoeia Dissolution Test Method 2 (Puddle Method) using 900 mL of water as a dissolution medium at 50 rpm, the dissolution rate of the active ingredient is 80% or less at a test time of 5 minutes, and 85% or more at a test time of 15 minutes, and preferably has performance such that the dissolution rate of the active ingredient is 70% or less at a test time of 5 minutes, and 90% or more at a test time of 15 minutes.

In a preferable aspect, the method for producing according to the present invention can be carried out by sequentially performing the step (1) of spraying a substantially spherical particle containing a saccharide and/or crystalline cellulose as a main component with a solution or a suspension containing the active ingredient and containing the binder, and then spraying a solution or a suspension of the disintegrant, the step (2) of spraying a water-dispersed ethylcellulose suspension containing the plasticizer, and the step (3) of adding the glidant and heating the resulting particle in the closed state under a condition of a temperature of 70 to 100°C for 15 to 180 minutes.

One aspect of the granule of the present invention is a granule obtained by a method including the above-described steps (1), (2), and (3).

In addition, one aspect of the granule of the present invention is a granule including a core particle containing an active ingredient having an unpleasant taste and including a film containing ethylcellulose in an amount of 2 to 12 wt% with respect to the core particle, wherein the core particle is coated with the film, the unpleasant taste of the active ingredient is masked, and immediate release of the active ingredient is maintained. Such a granule can be produced, for example, with the above-described method.

One aspect of the granule of the present invention is a granule including a core particle containing an active ingredient having an unpleasant taste and including a film containing ethylcellulose in an amount of 2 to 12 wt% with respect to the core particle, wherein the core particle is coated with the film, and when the granule is measured with the Japanese Pharmacopoeia Dissolution Test Method 2 (Puddle Method) using 900 mL of water as a dissolution medium at 50 rpm, the dissolution rate of the active ingredient is 80% or less at a test time of 5 minutes, and 85% or more at a test time of 15 minutes. Such a granule can be produced, for example, with the above-described method.

Examples of the active ingredient having an unpleasant taste that can be used in the granule of the present invention include the above-described active ingredients such as zinc acetate. Zinc acetate may be an anhydride or a hydrate, and is more preferably a dihydrate.

The particle size of the granule of the present invention can be appropriately selected and is not particularly limited, and can be, for example, about 200 to 700 µm. If a granule having such a particle size is used, a preparation can be obtained that is easy to take even for children and the elderly.

### Examples

The present invention will be described below with reference to specific embodiments, but the present invention is not limited to the embodiments, and it will be understood that various changes and modifications in the embodiments may be made by those skilled in the art without departing from the scope or gist of the invention defined in the appended claims.

### Example 1 and Comparative Example 1: Confirmation of Effect of Closed Condition

### (1) Preparation of Granule

Into a tumbling fluidized bed device, 1,300 g of spherical particles including sucrose and corn starch and having a particle size of 355 to 500 µm (product name: NONPAREIL (registered trademark)-101, manufactured by Freund Corporation) were charged, and the drug substance solution and the disintegrant suspension having the following compositions respectively were sprayed in this order under the condition of an air supply temperature of 65°C to obtain drug substance-coated base particles.

Drug substance solution: Solution prepared by dissolving 338.6 g of zinc acetate hydrate (C₄H₆O₄Zn·2H₂O) in 1,183 g of a 6% hydroxypropylcellulose solution

Disintegrant suspension: A suspended liquid obtained by adding 41.1 g of partly pregelatinized starch to 352 g of a 6% hydroxypropylcellulose solution

### (Example 1)

Onto 1,500 g of the resulting drug substance-coated base particles, 450 g of a 20% ethylcellulose suspension containing about 2% of triethyl citrate was sprayed, and the resulting particles were sieved using a 18 mesh sieve to obtain ethylcellulose-coated particles.

To the resulting ethylcellulose-coated particles, light anhydrous silicic acid was added and mixed at a content equivalent to 0.7%, and the resulting mixture was put in a closed container and heated under the condition of a temperature of 80°C for 2 hours (hereinafter, the resulting granule is referred to as "granule heated under the closed condition").

### (Comparative Example 1)

For comparison, the ethylcellulose-coated particles with light anhydrous silicic acid mixed at a content equivalent to 0.7% were put in an open container, and heated under the condition of a temperature of 80°C for 2 hours to obtain particles (hereinafter, referred to as "granule heated under the open condition").

### (2) Dissolution Test

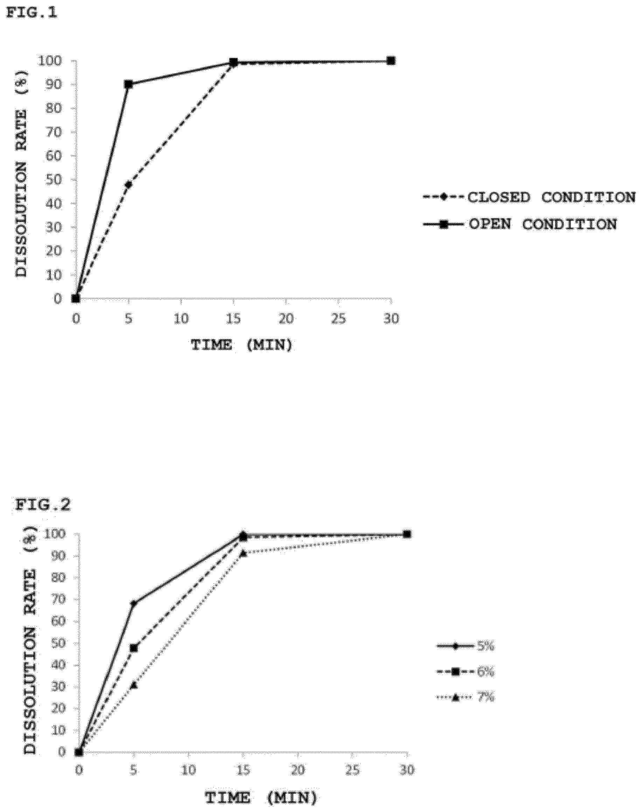

According to the Japanese Pharmacopoeia Dissolution Test Method 2 (Puddle Method, dissolution medium: water, dissolution medium amount: 900 mL, 50 rpm), the prepared granules (the granule heated under the closed condition and the granule heated under the open condition) were measured to determine the dissolution rates of the active ingredient at a test time of 5 minutes, 15 minutes, and 30 minutes. For the quantification, an ultraviolet-visible absorptiometer (detection reagent: 1-(2-hydroxycarbonylphenyl)-5-(2-hydroxy-5-sulfophenyl)-3-phenylformazan, measurement wavelength: 620 nm) was used. The results are shown in Fig. 1 (comparison between the dissolution rate of the active ingredient in the preparation heated under the closed condition and that in the preparation heated under the open condition) and Table 1 (results of the dissolution test).

**[Table 1]**

| Results of Dissolution Test (Dissolution Rate at Each Time Point) | | | | | |
|---|---|---|---|---|---|
| Time (min) | | 0 | 5 | 15 | 30 |
| Dissolution rate (%) | Closed condition | 0 | 47.9 | 98.6 | 100 |
| | Open condition | 0 | 90.0 | 99.3 | 100 |

As shown in Fig. 1 and Table 1, the dissolution rate at a test time of 15 minutes was almost 100% in both the granule heated under the closed condition and the granule heated under the release condition, and there was no difference between the granules. From these results, it was confirmed that immediate release of the active ingredient was sufficiently maintained in both the granule heated under the closed condition and the granule heated under the release condition. Meanwhile, the dissolution rates at a test time of 5 minutes showed that dissolution was suppressed in the granule heated under the closed condition more than in the granule heated under the released condition. These results indicate that by the heat treatment under the closed condition, dissolution of the active ingredient at an early stage after the administration is superiorly suppressed to mask the unpleasant taste effectively.

From the above-described results, it has been confirmed that a granule in which an unpleasant taste of an active ingredient is masked and immediate release of the active ingredient is maintained can be produced by using the method for producing of the present invention, that is, the method including the heat treatment under the closed condition in the coating step.

### Example 2: Coating Amount of Ethylcellulose

Granules heated under the closed condition were prepared in the same manner as in Example 1 except that the amount of a 20% ethylcellulose suspension containing about 2% of triethyl citrate sprayed onto the drug substance-coated base particles was adjusted so that the coating amount (the coating amount of ethylcellulose with respect to the drug substance-coated base particles) was 5, 6 and 7 wt%, respectively, and the dissolution rate of the active ingredient in each granule was confirmed with the same test method as in Example 1. The results are shown in Fig. 2 (the relationship between the coating amount of ethylcellulose and the dissolution rate of the active ingredient).

As shown in Fig. 2, the dissolution rate at a test time of 15 minutes was 85% or more in all of the granules having a coating amount described above. From these results, it was confirmed that the immediate release of the active ingredient was maintained in all of the granules. The dissolution rate at a test time of 5 minutes was as good as less than 70% in all of the granules having a coating amount described above. From these results, it was confirmed that the unpleasant taste was masked in all of the granules.

### Example 3: Examination of Addition Amount of Plasticizer

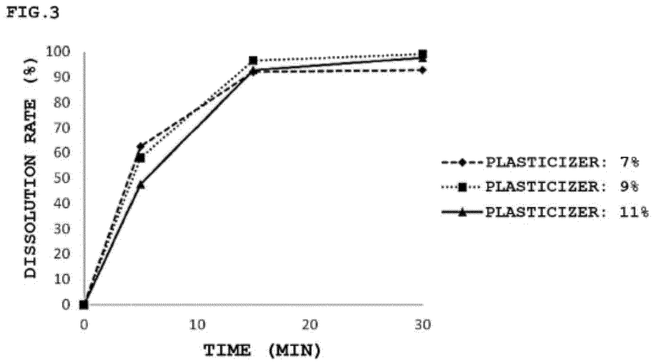

Granules heated under the closed condition were prepared in the same manner as in Example 1 except that the amount of the plasticizer blended in the ethylcellulose suspension was adjusted to 7, 9 and 11 wt%, respectively, with respect to ethylcellulose, and the dissolution rate of the active ingredient in each granule was confirmed with the same test method as in Example 1. The results are shown in Fig. 3 (the relationship between the addition amount of the plasticizer and the dissolution rate of the active ingredient).

As shown in Fig. 3, in the granules in which the amount of the plasticizer blended was 7 to 11% respectively, there was almost no difference between active ingredient dissolution behaviors.

### Industrial Applicability

According to the present invention, it is possible to provide a granule in which an unpleasant taste of an active ingredient is masked and immediate release of the active ingredient is maintained and to provide a method for producing the granule, and it is particularly possible to provide a granule that is further easy to take for patients of a wide age range from children to the elderly, and whose dose is easy to adjust.

## Claims

1. A method for producing a granule in which an unpleasant taste of an active ingredient is masked and immediate release of the active ingredient is maintained, the method comprising:
a step (1) of obtaining a core particle containing an active ingredient having an unpleasant taste;
a step (2) of coating a surface of the core particle obtained in the step (1) with ethylcellulose to obtain a particle; and
a step (3) of subjecting the particle obtained in the step (2) to a heat treatment in a closed state.

2. The method according to claim 1, wherein the step (1) is a step of coating a particle containing a saccharide and/or crystalline cellulose as a main component with the active ingredient.

3. The method according to claim 1 or 2, wherein the step (1) is a step of coating a particle containing a saccharide and/or crystalline cellulose as a main component with a binder and/or a disintegrant and with the active ingredient.

4. The method according to any one of claims 1 to 3, wherein the step (1) is a step of spraying a particle containing a saccharide and/or crystalline cellulose as a main component with a solution containing the active ingredient or with a suspension containing the active ingredient to coat the particle with the active ingredient.

5. The method according to any one of claims 1 to 4, wherein
the step (1) is a step of spraying a particle containing a saccharide and/or crystalline cellulose as a main component with a solution, the solution containing the active ingredient and a binder, or with a suspension, the suspension containing the active ingredient and a binder, to coat the particle with the active ingredient, and
further spraying the particle with a solution containing a disintegrant or with a suspension containing a disintegrant to coat the particle with the disintegrant.

6. The method according to any one of claims 2 to 5, wherein the particle containing a saccharide and/or crystalline cellulose as a main component has a substantially spherical shape.

7. The method according to claim 1, wherein the step (1) is a step of grinding a granulated product that includes the active ingredient having the unpleasant taste and a preparation aid to obtain particles, and sieving the particles to obtain particles having a certain particle size.

8. The method according to any one of claims 1 to 7, wherein the step (2) is a step of spray-coating the surface of the core particle obtained in the step (1) with a water-dispersed ethylcellulose suspension.

9. The method according to any one of claims 1 to 8, wherein the step (2) is a step of coating the surface of the core particle obtained in the step (1) with ethylcellulose and a plasticizer.

10. The method according to any one of claims 1 to 9, wherein the step (2) is a step of spray-coating the core particle obtained in the step (1) with a water-dispersed ethylcellulose suspension containing a plasticizer.

11. The method according to any one of claims 1 to 10, wherein a coating amount of the ethylcellulose in the step (2) is 5 to 9 wt% with respect to the core particle.

12. The method according to claims 11, wherein the coating amount of the ethylcellulose in the step (2) is 5 to 7 wt% with respect to the core particle.

13. The method according to any one of claims 1 to 12, wherein the step (3) is a step of subjecting the particle obtained in the step (2) to the heat treatment in the closed state under a condition of a temperature of 70 to 100°C for 15 to 180 minutes.

14. The method according to any one of claims 1 to 13, wherein the active ingredient having the unpleasant taste is zinc acetate.

15. The method according to any one of claims 1 to 14, wherein when an obtained granule is measured with the Japanese Pharmacopoeia Dissolution Test Method 2 (Puddle Method) using 900 mL of water as a dissolution medium at 50 rpm, a dissolution rate of the active ingredient is 80% or less at a test time of 5 minutes, and 85% or more at a test time of 15 minutes.

16. The granule in which an unpleasant taste of an active ingredient is masked and immediate release of the active ingredient is maintained, obtained by the method according to any one of claims 1 to 15.

17. A granule comprising: a core particle containing an active ingredient having an unpleasant taste; and a film containing ethylcellulose in an amount of 2 to 12 wt% with respect to the core particle, the core particle coated with the film, wherein the unpleasant taste of the active ingredient is masked, and immediate release of the active ingredient is maintained.

18. A granule comprising: a core particle containing an active ingredient having an unpleasant taste; and a film containing ethylcellulose in an amount of 2 to 12 wt% with respect to the core particle, the core particle coated with the film, wherein when the granule is measured with the Japanese Pharmacopoeia Dissolution Test Method 2 (Puddle Method) using 900 mL of water as a dissolution medium at 50 rpm, a dissolution rate of the active ingredient is 80% or less at a test time of 5 minutes, and 85% or more at a test time of 15 minutes.

19. The granule according to claim 17 or 18, wherein the active ingredient having the unpleasant taste is zinc acetate.
